(19) Europäisches Patentamt
European Patent Office
Office européen des brevets

(11) EP 1 211 237 A1

(12) EUROPEAN PATENT APPLICATION

(43) Date of publication:
**05.06.2002 Bulletin 2002/23**

(51) Int Cl.[7]: **C07C 67/14**, C07C 69/732, C07C 69/734

(21) Application number: **00310718.2**

(22) Date of filing: **01.12.2000**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Medicam Chemical & Pharmaceutical Co., Ltd.**
**Taipei Taiwan (CN)**

(72) Inventor: **Lee, Yean-Jang**
**Hsi-Tun Road Taichung, Taiwan (CN)**

(74) Representative: **Sexton, Jane Helen**
**J.A. KEMP & CO.**
**14 South Square**
**Gray's Inn**
**London WC1R 5JJ (GB)**

# (54) A one pot process for the preparation of caffeic acid ester derivatives

(57) The present invention relates to a one pot process for the preparation of caffeic acid ester derivatives of the formula I:

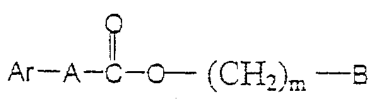

I

wherein

Ar is aryl, which is unsubstituted or substituted with halogen, hydroxy, $C_{1-6}$ alkyl or $C_{1-6}$ alkoxy,
A is $C_{1-6}$ alkylene, $C_{2-6}$ alkenylene or $C_{2-6}$ alkynylene,
m is a number from 0 to 6; and
B is $C_{1-6}$ alkyl or aryl, which is unsubstituted or substituted with halogen, hydroxy, $C_{1-6}$ alkyl or $C_{1-6}$ alkoxy;

which process comprises reacting, in a suitable solvent for esterification of a carboxylic acid derivative, a compound of the formula II:

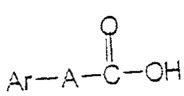

II

wherein

Ar and A are as defined above.

with a halogenating reagent and alcohol of the formula III:

$$H\text{-}(CH_2)_m\text{-}B \qquad III$$

wherein

EP 1 211 237 A1

m and B are as defined above.

## Description

The present invention relates to one pot processes for the preparation of caffeic acid ester derivatives.

Caffeic acid ester derivatives (e.g. caffeic acid phenylethyl ester (CAPE)), which are present in propolis, can be extracted by general known methods or synthesized by known chemical methods. For instance, Nakanishiet et al, (Experientia, 44:230-232, 1988) use methanesulfonic acid as a catalyst and heat caffeic acid and phenylethyl alcohol in benzene to reflux for 4 days to obtain a 40 % yield of the desired CAPE. Rao et al, (Chem. Biol. Interact., 84:277-290, 1992) use hexamethylphosphoric triamide (HMPA) as a solvent, 25% sodium hydroxide as a catalyst and replace (2-phenylethyl)benzene with phenylethyl alcohol to synthesize CAPE. The synthesis reaction is performed at room temperature for 2 days. Furthermore, Chen et al., (Cancer Lett, 108:211-214, 1996) use dicyclohexyl carbodiimide (DCC) as a catalyst to synthesize CAPE. The synthesis reaction is performed at room temperature for 2 days. In addition, the hydroxy on the phenyl ring of caffeic acid can be protected by a protecting group (e.g., biphenyl dichloromethane). The protected acid is esterified and deprotected by appropriate acids (e.g., acetic acid) to obtain CAPE. Alternatively, caffeic acid is reacted with a halogenating reagent (e.g., $SOCl_2$) and then the resultant product is reacted with phenylethyl alcohol to synthesize CAPE.

However, the above known methods have disadvantages including lower yields, longer reaction time, greater reactant amounts, more complex steps and undesired products. These methods do not fully satisfy the requirements of commercial production for caffeic acid esters.

It is an object of the present invention to provide a one pot process for the preparation of caffeic acid ester derivatives of the formula I:

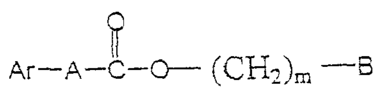

I

wherein

Ar is aryl, which is unsubstituted or substituted with halogen, hydroxy, $C_{1-6}$ alkyl or $C_{1-6}$ alkoxy,

A is $C_{1-6}$ alkylene, $C_{2-6}$ alkenylene or $C_{2-6}$ alkynylene,

m is a number from 0 to 6; and

B is $C_{1-6}$ alkyl or aryl, which is unsubstituted or substituted with halogen, hydroxy, $C_{1-6}$ alkyl or $C_{1-6}$ alkoxy,

which process comprises reacting, in a suitable solvent for esterification of a carboxylic acid derivative, a compound of the formula II:

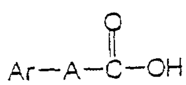

II

wherein

Ar is aryl, which is unsubstituted or substituted with halogen, hydroxy, $C_{1-6}$ alkyl or $C_{1-6}$ alkoxy,

A is $C_{1-6}$ alkylene, $C_{2-6}$ alkenylene or $C_{2-6}$ alkynylene, with a halogenating reagent and an alcohol of the formula III:

$$HO\text{-}(CH_2)_m\text{-}B \qquad III$$

wherein

m is a number from 0 to 6; and

B is $C_{1-6}$ alkyl or aryl, which is unsubstituted or substituted with halogen, hydroxy, $C_{1-6}$ alkyl or $C_{1-6}$ alkoxy.

The term "halogen", as used herein, refers to fluorine, chlorine, bromine or iodine.

The term "aryl", as used herein, refers to an aromatic, mono- to tri-nuclear cyclic system with 6-14 members such as phenyl, naphthyl and anthryl which may be substituted, preferably with one to four substituents, which are preferably independently hydroxy, $C_{1-6}$ alkyl, $C_{1-6}$ alkoxy or halogen. The preferred aryl is phenyl unsubstituted or substituted with one to three substituents which are independently hydroxy, $C_{1-6}$ alkyl, $C_{1-6}$ alkoxy or halogen. The most preferred aryl is 3,4-dihydroxyphenyl or 3,4-dimethoxyphenyl, especially 3,4-dihydroxyphenyl.

The term "halogenating reagent", as used herein, refers to the reagents halogenating carboxylic acid to acid halide. The preferred reagents are thionyl chloride ($SCOl_2$), oxalyl chloride (ClCOCOCl), phosphoric chloride and phosphorous chloride.

The term "suitable esterifying solvent", as used herein, refers to the solvents facilitating the esterification between the reactants in the process of the invention. For instance, an aprotic solvent such as methyl chloride or benzene; or a protic solvent such as trichloromethane, acetone, acetonitrile, nitrobenzene, THF, ether, dioxane, DMF and DMSO can applied. The polar solvent is preferred. The most preferred solvent is nitrobenzene.

The present invention relates to a one pot processes for the preparation of caffeic acid ester derivatives of formula I wherein the reaction steps are as follows:

$$\text{Ar-A-CO-OH} + \text{HO-}(CH_2)_m\text{-B} \xrightarrow[\text{suitable esterifying solvant}]{\text{halogenating reagent}} \text{Ar-A-C-O-O-}(CH_2)_m\text{-B} \qquad \text{II}$$

wherein Ar, A, m and B are as defined as above.

Typically the amount of alcohol is from 0.25 to 4 mols per mol of carboxylic acid, preferably 0.5 to 2 mols of alcohol per mol of carboxylic acid, more preferably 0.8 to 1.2. Most preferably the molar quantities of carboxylic acid and alcohol are approximately equivalent.

Typically the amount of halogenating reagent is from 0.25 to 4 mols per mol of carboxylic acid, preferably 0.5 to 2 mols of halogenating reagent per mol of carboxylic acid, more preferably 0.8 to 1.2. Most preferably the molar quantities of halogenating reagent and carboxylic acid are approximately equivalent.

The above reaction can be accomplished at room temperature or at elevated temperature by heating to obtain rapidly the final ester.

The temperature at which the reaction is carried out is preferably 80°C to 120°C, more preferably 90°C to 120°C. A reaction temperature of approximately 90°C is most preferred. The reaction time is generally one to six hours, preferably one to four hours, more preferably one to three hours eg. about two hours.

Surprisingly, the preparation processes of the invention can be performed in one pot. The alcohol amounts essential for the processes are less than those of the known processes. Furthermore, the reaction time of the inventive processes may be reduced to eg. 2 hours and the yield of the inventive processes is more than 50%. Therefore, the process of the invention solves the disadvantages of the known processes of preparing caffeic acid ester derivatives. In addition, the process of the invention is cheaper in cost than the known processes.

To facilitate the understanding of purpose, methods, characteristics and principles of the invention, the following examples illustrate various aspects of the present invention.

Examples

Example 1

A solution of caffeic acid (1.05 g, 5.8 mmol), $SOCl_2$ (0.43 ml, 5.8 mmol), and phenylethyl alcohol (0.70 ml, 5.8 mmol) in nitrobenzene (20 ml) was heated for 2 hours. The resulting solution was subjected to column chromatography (Silica, ether : hexane = 1 : 1) to give the desired CAPE (1.42 g, yield :60 %).
mp 127-128 °C.
$^1$H NMR (400 MHz, $CDCl_3$): δ 3.02 (2 H, t, *J*= 7.2 Hz), 4.42 (2 H, t, *J*=7.2 Hz), 5.98 and 6.14 (2 H, brs), 6.25 (1 H, d, *J*= 16 Hz), 6.87 (1 H, d, *J*= 8 Hz), 6.99 (1 H, dd, *J*= 8, 2 Hz), 7.09 (1 H, d, *J*= 2 Hz), 7.23-7.34 (5 H, m), 7.57 (1 H, d, *J*=16.0Hz).
$^{13}$C NMR (100 MHz, $CDCl_3$): δ 35.3, 65.2, 114.3, 115.2, 115.4, 122.4, 126.5, 127.3, 128.4, 128.8, 137.6, 143.7, 145.1, 146.3, 167.6.
EIMS m/z (rel. int): 284 $[M]^+$ (21), 180(100), 163(44).
HRMS: Calc. for $C_{17}H_{16}O_4$: 284.1048. Found: 284.1047. Anal. Calcd for $C_{17}H_{16}O_4$: C, 71.82; H, 5.67; O, 22.51. Found: C, 71.55; H, 5.60; O, 22.75.

**Example 2:** Comparison between the processes of the invention and the conventional processes

Comparative Example A

According to the results of Nakanishiet et al, (Experientia, 44:230-232, 1988), the use of methanesulfonic acid as a catalyst; caffeic acid and phenylethyl alcohol in benzene were heated to reflux for 4 days to obtain 40 % yield of CAPE.

Comparative Example B

The caffeic acid (0.5 g, 2.7 mmole) and dicyclohexyl carbodiimide (DCC) (0.372 g, 3.0 mmol) were placed together with dimethylaminopyridine (DMAP) (0.459 g, 2.2 mmol) into a 25 ml reaction bottle. 10 ml DMF/$CH_2Cl_2$ (1: 1) solution was added to the mixture. Phenylethyl alcohol (1 ml, 8.3 mmol) was slowly added to the reaction bottle. The reaction mixture was stirred at room temperature. The precipitate was produced. The reaction liquid was filtrated, taken and chromatographed by TLC (diethyl ether : hexane = 2: 1). The products were separated through a silica column and then were eluted with diethyl ether : hexane = 2 : 1. The residue was concentrated to obtain a 46 % yield of CAPE.

Comparative Example C

Caffeic acid (0.50 g, 2.8, mmol) was placed together with dichlorodiphenylmethane (0.54 ml, 2.8 mmol, 1.0 equiv.) into a 25 ml, two-necked, round-bottomed flask under a nitrogen atmosphere. The mixture was then heated to 170-180 °C (oil bath temperature) while being stirred under nitrogen reflux and was maintained at that temperature for 15 minutes. The flask was removed from the oil bath and allowed to cool to ambient temperature. The dark brown residue was dissolved in dichloromethane, applied to a 3x30 cm $SiO_2$ column, and eluted with diethyl ether/petroleum ether (1:1). The crude product was recrystallized from cyclohexane as a white solid (0.53 g). The purified acid was added to excess $SOCl_2$, used as solvant, and was heated to reflux for 2 hours. The residue was concentrated by a reducing pressure machine and $SOCl_2$ was removed. The concentrated acid chloride in dichloromethane was slowly added to phenylethyl alcohol (183 μl, 15.40 mmole) and pyridine (121 μl, 15.40 mmole) and then was stirred at room temperature for 2 hours. The reaction liquid was taken and chromatographed by TLC (diethyl ether : hexane = 2: 1). The products were separated through a silica column and then were eluted with diethyl ether : hexane = 2 : 1. The residue was concentrated to a white solid, which was reacted with acetic acid to obtain a 12% yield of CAPE.

Comparative Example D

The caffeic acid (500 mg, 2.77 mmole) in excess $SOCl_2$ was heated to reflux for 2 hours. $SOCl_2$ was removed and the residue was concentrated in a vacuum. The concentrated acid chloride in 10 ml nitrobenzene was slowly added to phenylethyl alcohol (1.65 ml, 13.8 mmole) and then was heated at 50 °C for 2 hours. The reaction liquid was taken and chromatographed by TLC (diethyl ether : dichloromethane : hexane = 2: 2 :1) to identify the formation of the products, which were separated through a silica column. Dichloromethane was firstly used as eluant to remove nitrobenzene and then, the product was collected by eluting with diethyl ether : dichloromethane : hexane = 2 : 2 : 1. The residue was concentrated to a white solid, which was recrystalized by dichloromethane/hexane to obtain a 50% yield of CAPE.

The comparative results are as follows:

| Examples | alcohol amounts | reaction time | cost/g | CAPE yield |
|---|---|---|---|---|
| Comparative Example A | 5 equivalent | 3 days | larger than NT$328 | 0-40% |
| Comparative Example B | 1 equivalent | 2 days | NT$350 | 46% |
| Comparative Example C | 1 equivalent | 8 hours | larger than NT$1000 | 12% |
| Comparative Example D | 5 equivalent | 4 hours | NT$500 | 50% |
| Example 1 | 1 equivalent | 2 hours | NT$250 | 60% |

In view of the above table, the processes of the invention are superior in alcohol amounts, reaction time, cost and CAPE yield than the known processes.

## Claims

**1.** A one pot process for preparing a caffeic acid ester derivative of the formula I

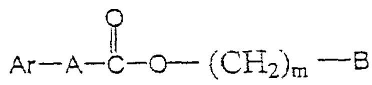

I

wherein
Ar is aryl, which is unsubstituted or substituted with halogen, hydroxy, $C_{1-6}$ alkyl or $C_{1-6}$ alkoxy,
A is $C_{1-6}$ alkylene, $C_{2-6}$ alkenylene or $C_{2-6}$ alkynylene,
m is a number from 0 to 6; and
B is $C_{1-6}$ alkyl or aryl, which is unsubstituted or substituted with halogen, hydroxy, $C_{1-6}$ alkyl or $C_{1-6}$ alkoxy;
which process comprises reacting, in a suitable solvent for esterification of a carboxylic acid derivative, a compound of the formula II:

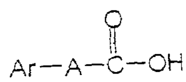

wherein
Ar is aryl, which is unsubstituted or substituted with halogen, hydroxy, $C_{1-6}$ alkyl or $C_{1-6}$ alkoxy;
A is $C_{1-6}$ alkylene, $C_{2-6}$ alkenylene or $C_{2-6}$ alkynylene, with a halogenating reagent and alcohol of the formula III:

$$H\text{-}(CH_2)_m\text{-}B \qquad III$$

wherein
m is a number from 0 to 6, and
B is $C_{1-6}$ alkyl or aryl, which is unsubstituted or substituted with halogen, hydroxy, $C_{1-6}$ alkyl or $C_{1-6}$ alkoxy.

**2.** The process according to claim 1, wherein the halogenating reagent is thionyl chloride or oxalyl chloride.

**3.** The process according to claim 1 or 2, wherein the reaction is performed under at elevated temperature.

**4.** The process according to any one of claims 1 to 3 wherein the suitable esterifying solvent is an aprotic solvent.

**5.** The process according to claim 4, wherein the aprotic solvent is methyl chloride or benzene.

**6.** The process according to any one of claims 1 to 3 wherein the suitable esterifying solvent is a protic solvent.

**7.** The process according to claim 6, wherein the protic solvent is trichloromethane, acetone, acetonitrile, nitrobenzene, THF, ether, dioxane, DMF or DMSO.

**8.** The process according to claim 7, wherein the protic solvent is nitrobezene.

**9.** The process according to any one of the preceding claims wherein Ar is phenyl, naphthyl or anthryl, optionally substituted by one to four substituents which are independently hydroxy or $C_{1-6}$ alkoxy.

**10.** The process according to claim 9, wherein Ar is phenyl substituted by one to three hydroxy groups.

**11.** The process according to claim 10, wherein Ar is 3,4-dihydroxyphenyl, A is $C_2$ alkenylene, m is 2 and B is phenyl.

**12.** The process according to claim 9, where Ar is phenyl substituted by one to three $C_{1-6}$ alkoxy groups.

**13.** The process according to claim 12, wherein Ar is 3,4-dimethoxyphenyl.

European Patent Office

# EUROPEAN SEARCH REPORT

Application Number
EP 00 31 0718

| Category | Citation of document with indication, where appropriate, of relevant passages | Relevant to claim | CLASSIFICATION OF THE APPLICATION (Int.Cl.7) |
|---|---|---|---|
| A | HIROYKI NAKATA ET AL.: "A New Phenolic Compound from Heracleum lanatum Michx.var.nippinicum Hara. II" CHEMICAL AND PHARMACEUTICAL BULLETIN., vol. 30, no. 12, December 1982 (1982-12), pages 4554-4556, XP002165977 PHARMACEUTICAL SOCIETY OF JAPAN. TOKYO., JP ISSN: 0009-2363 * page 4556, paragraph 3 * | 1 | C07C67/14<br>C07C69/732<br>C07C69/734 |
| A | L.H.KLEMM ET AL.: "Electroreduction of alpha,beta-Unsaturated Esters. II. Syntheses of 2,3-Diaryl-5-oxocyclopentane-1-carboxylates by Hydrodimerization of Cinnamates" JOURNAL OF ORGANIC CHEMISTRY., vol. 38, no. 19, 21 September 1973 (1973-09-21), pages 3390-3394, XP002165978 AMERICAN CHEMICAL SOCIETY. EASTON., US ISSN: 0022-3263 * page 3392, right-hand column, paragraph 2 * | 1 | TECHNICAL FIELDS SEARCHED (Int.Cl.7)<br>C07C |

The present search report has been drawn up for all claims

| Place of search | Date of completion of the search | Examiner |
|---|---|---|
| THE HAGUE | 25 April 2001 | Kinzinger, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)